Europäisches Patentamt

European Patent Office

Office européen des brevets

(19)

(11) Numéro de publication : **0 466 560 A1**

# (12) DEMANDE DE BREVET EUROPEEN

(21) Numéro de dépôt : **91401853.6**

(22) Date de dépôt : **04.07.91**

(51) Int. Cl.⁵ : **C12M 1/12, C12M 1/36**

(30) Priorité : **11.07.90 FR 9008807**

(43) Date de publication de la demande :
**15.01.92 Bulletin 92/03**

(84) Etats contractants désignés :
**AT BE CH DE DK ES FR GB GR IT LI LU NL SE**

(71) Demandeur : **LACTO LABO**
**Zone d'Activités de Buxières, B.P. 10**
**F-86220 Dange-Saint-Romain (FR)**

(72) Inventeur : **Bibal, Bernard**
**21, Rue Frédéric Mistral**
**F-12850 Sebazac-Concoures (FR)**
Inventeur : **Goma, Gérard**
**20, Rue de Salas**
**F-31520 Ramonville St-Agne (FR)**
Inventeur : **Pareilleux, Alain**
**24, Rue de l'ancienne Batterie**
**F-31520 Ramonville St-Agne (FR)**

(74) Mandataire : **Dutruc-Rosset, Marie-Claude et
al**
**RHONE-POULENC CHIMIE Direction des
Brevets 25, Quai Paul Doumer**
**F-92408 Courbevoie Cédex (FR)**

(54) **Procédé de fabrication de microorganismes.**

(57)    L'invention concerne un procédé de fabrication de microorganismes producteurs de leur propre inhibiteur de croissance, en particulier des bactéries lactiques, ledit procédé permettant d'améliorer et de contrôler l'activité cellulaire spécifique et la productivité cellulaire des microorganismes.
Un objet de l'invention consiste en des microorganismes à activité cellulaire spécifique et productivité cellulaire améliorées.

EP 0 466 560 A1

FIG.1

La présente invention concerne un procédé de fabrication de microorganismes producteurs de leur propre inhibiteur de croissance, ainsi que des microorganismes dont l'activité cellulaire spécifique et la productivité cellulaire sont améliorées. Lesdits microorganismes peuvent être en particulier des bactéries lactiques.

La fabrication de microorganismes s'effectue de manière classique dans un milieu de culture contenant des substrats nutritifs dilués dans de l'eau. Ce milieu de culture est tout d'abord ensemencé en microorganismes qui se développent ainsi en milieu quasi fermé. Cette culture est dite en batch. La culture en batch présente l'avantage d'être simple, mais d'une faible efficacité. En effet, il existe des facteurs limitant, soit dans les substrats nutritifs, soit dans les inhibiteurs produits par les microorganismes dont l'accumulation dans le milieu de fermentation réduit le taux de croissance des microorganismes et provoque un arrêt de la fermentation. Parallèlement, le rendement en biomasse formée par rapport aux substrats nutritifs introduits notamment par rapport aux sources carbonées et azotées, diminue progressivement.

La demanderesse a également pu constater que des microorganismes obtenus selon un tel procédé en batch, présentaient une activité cellulaire spécifique et une productivité cellulaire insuffisantes et non contrôlables, c'est à dire que d'une culture en batch à l'autre, les microorganismes obtenus peuvent présenter une activité cellulaire spécifique et une productivité cellulaire différente. On entend par activité cellulaire spécifique, l'expression de la capacité d'une quantité donnée de microorganismes à exécuter la fonction qu'on leur a assignée. La productivité cellulaire est égale à l'activité cellulaire spécifique multipliée par la concentration bactérienne et divisée par le temps de fermentation. Ainsi l'activité cellulaire spécifique et la productivité celllulaire sont propres à chaque type de microorganismes.

L'activité spécifique cellulaire et la productivité cellulaire ne sont donc comparables que d'un microorganismes d'un certain type à un microorganisme du même type. La détermination de l'activité cellulaire spécifique et de la productivité cellulaire d'un microorganisme ne peut donc se faire que selon une méthode établie propre à chaque type de microorganisme.

Les techniques permettant de déterminer l'activité cellulaire spécifique et la productivité cellulaire d'un microorganisme donné sont généralement bien connues de l'homme de l'art. Toutefois dans le cas d'un microorganisme pour lequel de telles méthodes n'ont pas encore été mises au point, il est tout à fait à la portée de l'homme de l'art d'établir une telle méthode. Un exemple de détermination de l'activité cellulaire spécifique et de la productivité cellulaire est donné ci-après pour ce qui concerne les bactéries lactiques.

Afin de pallier aux inconvénients de la culture batch, il a été proposé de préparer des microorganismes en continu, c'est à dire par apport régulier en substrats nutritif parallèlement à une élimination des inhibiteurs du milieu de fermentation. L'élimination des inhibiteurs a été réalisée selon différents moyens, par exemple par dyalise ou par ultrafiltration.

Le brevet européen 65 895 décrit un procédé de fabrication de microorganismes en recyclage total de la biomasse, qui met en oeuvre une élimination des inhibiteurs de croissance par ultrafiltration. Toutefois un tel procédé, s'il permet d'atteindre de fortes concentrations en microorganismes, présente des inconvénients.

Ainsi, l'augmentation de la biomasse dans le fermenteur rend la conduite du procédé délicate, ceci notamment par le fait que la viscosité du milieu augmente fortement.

La demanderesse a également pu constater qu'un procédé à recyclage total de biomasse ne permettait pas l'obtention de microorganismes dont l'activité cellulaire spécifique et la productivité cellulaire étaient améliorées ou contrôlables.

Afin d'obvier aux inconvénients de l'art antérieur, la demanderesse a mis au point un procédé de fabrication de microorganismes constituant un premier objet de la présente invention. Ce procédé permet notamment d'améliorer les rendements en biomasse par rapport aux substrats nutritifs et notamment par rapport aux sources carbonées et azotées constituants du milieu de culture mise en oeuvre. Il autorise également une conduite du système simplifié. Mais surtout, il permet d'obtenir des microorganismes dont l'activité cellulaire spécifique et la productivité cellulaire sont non seulement améliorées mais surtout contrôlées, c'est à dire, que l'on peut fabriquer des microorganismes dont l'activité cellulaire spécifique et la productivité cellulaire peuvent être déterminées par avance et restent constantes d'un procédé de fabrication à l'autre. Un tel procédé permet alors à l'utilisateur des microorganismes fabriqués de prévoir la dose nécessaire en dix microorganismes en vue de ses applications ultérieures. Le procédé selon l'invention peut être en particulier appliqué à la fabrication de bactéries lactiques.

Un autre objet de l'invention consiste en des microorganismes dont l'activité cellulaire spécifique et la productivité cellulaire sont améliorées. De tels microorganismes peuvent notamment être des bactéries lactiques.

la présente invention concerne donc un procédé de fabrication de microorganismes producteurs de leur propre inhibiteur de croissance, selon lequel on fait fermenter un milieu de culture par lesdits microorganismes de manière à obtenir un milieu de fermentation, on sépare par un procédé physique de séparation au moins une partie dudit inhibiteur du milieu de fermentation, et on recycle le retentat ledit procédé étant caractérisé en ce que lorsque le taux de croissance desdits microorganismes atteint une valeur égale à la valeur d'un taux de croissance, mesurée en régime permanent, correspondant à une activité cellulaire spécifique des microorganismes qui a été prédéterminée, on purge le milieu de fermentation selon un taux de purge supérieur ou égal audit taux de croissance.

Les autres caractéristiques et avantages de l'invention sont détaillés ci-dessous.

Les dessins ci-joints sont brièvement commentés :

– La figure 1 est une vue schématique d'une installation de fabrication de bactéries lactiques selon le procédé de l'invention

– La figure 2 est un diagramme montrant la relation entre l'activité cellulaire spécifique d'une souche Streptococcus cremoris par rapport à son taux de croissance.

– La figure 3 est un diagramme représentant la concentration en acide lactique en fonction de la conductivité mesurée.

– La figure 4 représente le rendement en biomasse d'une souche Streptococcus cremoris par rapport au sucre du milieu de culture, en fonction du taux de purge.

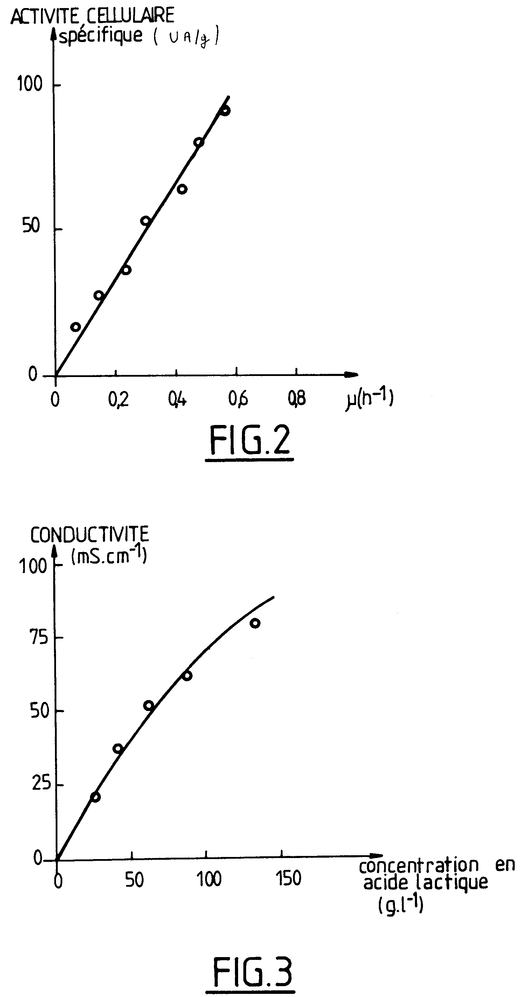

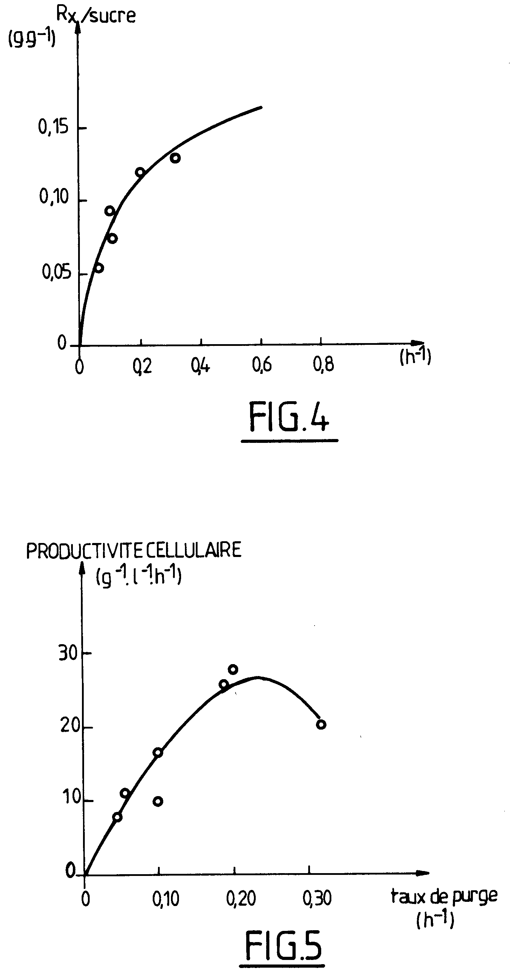

La souche Streptococcus cremoris de départ ayant servi à l'élaboration des figures 2 à 4 est une souche conservée dans la collection de souches de la société Lacto Labo sous le nom de code Sc 301. Cette souche est commercialisée par la société Lacto Labo sous forme d'un levain denommé SM 259.

Le taux de croissance qui indique le taux de purge à mettre en oeuvre dans le cadre de la présente invention est donc fixée par rapport à une activité cellulaire spécifique prédéterminée. La demanderesse a en effet pu constater que, d'une manière surprenante, l'activité cellulaire spécifique des microorganismes était liée à leur taux de croissance. La figure 2 illustre cette relation dans le cas de ladite souche Streptococcus cremoris de la société Lacto Labo.

Parallèlement, la demanderesse a pu constater qu'en purgeant le milieu de fermentation selon l'invention, il était possible d'imprimer au microorganisme une activité cellulaire spécifique choisie par le fabricant. De ce fait, pour fabriquer des microorganismes ayant une forte activité cellulaire spécifique, cette dernière pouvant par ailleurs être contrôlée, il suffit de purger le milieu de fermentation selon un taux de purge supérieur ou égal au taux de purge correspondant à l'activité cellulaire spécifique prédéterminée et recherchée desdits microorganismes.

Le régime permanent est obtenu quand la concentration en biomasse, en substrats nutritifs et produits inhibiteurs, présents dans le milieu de fermentation sont sensiblement constants.

l'activité cellulaire spécifique d'un type donné de microorganisme, lui est propre. De ce fait, il convient pour chaque type de microorganisme de déterminer la relation existant entre son activité cellulaire spécifique et son taux de croissance mesuré en régime permanent. Cette détermination peut se faire par tout moyen. Toutefois un moyen simple consiste à fermenter un milieu de culture par un microorganisme, dans un chemostat, c'est à dire un fermenteur de volume V, alimenté en continu par du milieu de culture selon un débit Q et dont on soustrait en continu du milieu de fermentation sous un même débit Q. Le débit Q est, dans des conditions de régime permanent, directement proportionnel aux taux de croissance $\mu$ selon la formule $\mu = Q/V$.

En faisant varier le débit Q on peut donc fixer différents taux de croissance.

A chaque fois que l'on a fait varier le débit Q, et après avoir atteint le régime permanent, on prélève des microorganismes dont on mesure l'activité cellulaire spécifique. Ainsi, on obtient pour chaque type de microorganismes que l'on veut fabriquer la relation entre son taux de croissance et son activité cellulaire spécifique. La figure 2 représente l'activité cellulaire spécifique, exprimée en $g^{-1}$, de ladite souche Streptococcus cremoris en fonction de son taux de croissance.

La détermination de l'activité cellulaire spécifique d'un microorganisme donné se fait selon une méthode qui est propre audit microorganisme. On donne à titre d'exemple, une méthode de détermination de l'activité cellulaire spécifique des bactéries lactiques. Cette méthode consiste dans un premier temps, à déterminer un certain volume $V_o$ de milieu de fermentation dans lequel est fabriqué le microorganisme ; ledit volume $V_O$ est celui nécessaire pour produire 75° dornic dans 10 ml de lait à 10 % en extrait sec, pendant 15 heures à 21°C. Un degré dornic est défini comme étant égal à 0,1 g d'acide lactique par litre de lait.

Le volume $V_O$ correspond à une unité d'activité (UA).

On détermine ensuite le nombre d'unité d'activité par litre du milieu de fermentation.

On peut alors déterminer l'activité cellulaire spécifique comme étant le nombre d'unité d'activité par litre de milieu de fermentation divisée par la concentration bactérienne du milieu de fermentation l'activité cellulaire spécifique est exprimée en UA/g.

Lorsqu'on purge le milieu de fermentation, il faut bien comprendre qu'on soutire hors du dispositif de fermentation une partie du milieu de fermentation, c'est à dire les substrats nutritifs en solution aqueuse formant le milieu de culture, ainsi que les microorganismes qui y sont contenus. Le taux de purge, exprimé en $h^{-1}$, est égal au rapport entre le débit auquel on purge le milieu de fermentation et le volume du milieu de fermentation.

Dans le cadre de la présente invention il suffit que le taux de purge soit supérieur ou égal au taux de croissance prédéterminé des microorganismes. Toutefois, la valeur maximale du taux de purge doit de préférence être inférieur au taux de croissance maximun théorique μ max des microorganismes que l'on veut fabriquer, de sorte à éviter de "laver" le dispositif contenant le milieu de fermentation. Avantageusement, le taux de purge est choisi à une valeur comprise entre 10 et 80 % du taux de croissance maximun théorique, de préférence entre 15 et 50 % du taux de croissance maximun théorique des microorganismes à fabriquer. Le taux de croissance maximun théorique peut être évalué pour chaque type de microorganisme selon une méthode classique, connue de l'homme de l'art.

Il peut arriver qu'avec certains microorganismes, cultivés dans un réacteur et dans des conditions particulières, le taux de croissance maximun théorique ne puisse être atteint. Dans ce cas, le taux de purge peut être choisi à une valeur comprise entre 15 et 80 % du taux de croissance maximun expérimental, plus préférentiellement entre 20 et 50 % du taux de croissance maximun expérimental. le taux de croissance maximun expérimental étant le taux de croissance maximun que peuvent atteindre lesdits microorganismes avec les conditions expérimentales choisies. Un tel taux de croissance maximun peut être défini par des méthodes classiques pour l'homme de l'art.

D'une manière avantageuse, on ne purge le milieu de fermentation selon l'invention que lorsque le taux de croissance prédéterminé est atteint, le taux de croissance étant déterminé par la mesure en continu de la concentration en inhibiteurs. Cette mesure peut être effectuée par exemple selon la méthode décrite ci-dessous dans le cas particulier des bactéries lactiques.

Au cours du procédé de fabrication selon l'invention, on maintient généralement constant le volume du milieu de fermentation par ajout de milieu de culture. Généralement, le pH du milieu de fermentation est maintenu à une valeur comprise entre 5 et 8.

Le pH peut être maintenu à une valeur désirée au moyen d'une base, en particulier d'une base minérale. La base minérale utilisée peut être de la soude, de la potasse, de l'ammoniac gazeux ou une solution aqueuse d'ammoniaque. L'ammoniac gazeux et l'ammoniaque en solution aqueuse sont préférés. L'ammoniac gazeux est tout particulèrement préféré.

D'une manière avantageuse, avant de procéder à la purge du milieu de fermentation selon l'invention, on peut fermenter le milieu de culture par les microorganismes à fabriquer en batch. De cette façon, il est possible d'atteindre plus rapidement le taux de croissance correspondant à celui de l'activité cellulaire spécifique prédéterminée.

En outre, et éventuellement, après avoir fermenté le milieu de culture en batch, on peut déjà séparer par un procédé physique de séparation au moins une partie dudit inhibiteur de croissance du milieu de fermentation avant de purger le milieu de fermentation. Cette séparation peut aussi, au cas où l'inhibiteur est trop concentré dans le milieu de fermentation, permettre d'atteindre plus rapidement et plus sûrement, le taux de croissance correspondant à celui de l'activité cellulaire prédéterminée.

Dans le cadre de l'invention, ledit procédé physique de séparation peut notamment consister en une ultrafiltration, une décantation, une centrifugation ou une dialyse. Toutefois l'ultrafiltration est préférée. Elle peut être mise en oeuvre au moyen d'un ou de plusieurs cellules d'ultrafiltration disposées en parallèle. Quelque soit le procédé de séparation utilisé, on entend par retentat dans le cadre de la présente invention, la fraction du milieu de fermentation recyclée. On entend par perméat la fraction du milieu de fermentation qui au cours du procédé physique de séparation n'est pas recyclée et qui est exempte ou quasiment exempte de microorganismes ou de débris microbiens.

La séparation de l'inhibiteur de croissance se fait selon un taux de dilution D.
Lorsqu'un tel procédé physique de séparation est mis en oeuvre, on peut purger le milieu de fermentation selon l'invention, en continu dès lors que la concentration de l'inhibiteur de croissance dans ledit milieu de fermentation est sensiblement constante. Généralement une telle concentration constante est atteinte après 3 temps de séjour liquide, plus généralement entre 3 et 10 temps de séjour liquide. Le temps de séjour liquide est exprimé en heures et correspond à l'inverse du taux de dilution D.
Le procédé selon l'invention peut être appliqué à tout microorganisme producteur de son propre inhibiteur de croissance.

On peut citer en particulier les microorganismes dont l'inhibiteur de croissance est constitué par un produit comme l'acide propionique notamment produit par des propionibactérium, l'acide acétique obtenus par exemple par des acétobacters, ou encore d'autres acides organiques comme les acides lactiques, citrique, itaconique, butyrique ou des alcools comme l'éthanol.

Le procédé selon l'invention s'applique tout particulièrement à la fabrication de bactéries lactiques.

Les bactéries lactiques forment un groupe non-taxonomique de bactéries Gram[+], ne formant pas de spores, qui peuvent fermenter des glucides solubles dans l'eau pour produire de l'acide lactique en tant que produit final, de manière prépondérante. Ces bactéries sont utilisées notamment dans les industries laitières, fromagères, de la salaison, en tant que probiotique, c'est à dire en tant qu'apport au maintien et à l'enrichissement de la flore intestinale humaine ou animale, ou en tant qu'agent d'ensilage. Ces bactéries appartiennent plus particulièrement aux genres Lactobacillus, Streptococcus, Leuconostoc et, Pediococcus. Les bactéries lactiques appartenant au genre Streptococcus sont préférées. La taxonomie de ces microorganismes a été décrite dans "Bergey's Manual of Systematic Bacteriology" (Sneath et al. 1986).

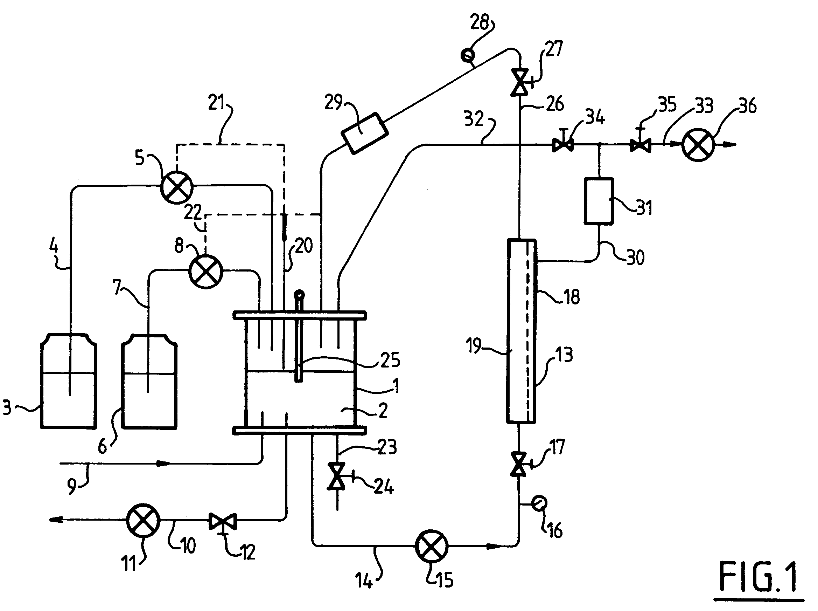

Le procédé de fabrication de bactéries lactiques selon l'invention peut être mis en oeuvre au moyen d'un dispositif tel que celui représenté à la figure 1. Bien entendu, ce dispositif peut être utilisé pour la fermentation d'autres microorganismes.

Ce dispositif comporte un fermenteur 1 contenant le milieu de fermentation 2, et est relié :

– à un réservoir 3 de source carbonée en solution aqueuse, par une conduite 4 équipée d'une pompe d'alimentation 5

– à un réservoir 6 de source azotée en solution aqueuse, par une conduite 7 équipée d'une pompe d'alimentation 8

– à un réservoir d'ammoniac (non représenté) par une conduite 9

– à une conduite de purge 10, munie d'une pompe de purge 11 et d'une vanne 12

– à une cellule ultrafiltration 18 par une conduite 14, équipé d'une pompe de recirculation 15, d'un manomètre 16 et d'une vanne 17.

la cellule d'ultrafiltration comporte un coté aval 18 et un coté amont 19.

le fermenteur 1 comporte également :

– un dispositif de mesure de niveau 20 relié à la pompe d'alimentation 5 et à la pompe d'alimentation 8 par deux dispositifs électriques, respectivement 21 et 22.

– une conduite d'échantillonage 23 munie d'une vanne 24.

– et un dispositif de mesure du pH 25 du milieu de fermentation.

le coté amont 19 de la cellule d'ultrafiltration 13 est relié au fermenteur par une conduite 26 équipée d'une vanne 27, d'un manomètre 28 et d'un échangeur thermique 29.

Le coté aval 18 de la cellule d'ultrafiltration 13 est munie d'une conduite 30 équipée d'une sonde conductimétrique 31. après la sonde conductimétrique 31, la conduite 30 se divise en deux conduites 32 et 33.

La conduite 32 est équipée d'une vanne 34 et, reliée au fermenteur 1.
La conduite 33 est équipée d'une vanne 35 et d'une pompe 36.

Le mode de fonctionnement du dispositif qui vient d'être décrit est le suivant :

au début d'une opération de fabrication de bactéries lactiques, on introduit dans le fermenteur 1 un volume de milieu de culture constitué pour une partie de la source carbonée en solution aqueuse provenant du réservoir 3, et pour une autre partie de la source azotée en solution aqueuse provenant du réservoir 6, puis un inoculum de la bactérie lactique à fabriquer. Dans le cas présent les sources carbonées et les sources azotées sont contenues dans des réservoirs distincts ce qui a notamment pour avantage de faciliter la stérilisation desdites sources. Toutefois on peut utiliser un réservoir unique contenant à la fois la source carbonée et la source azotée en solution aqueuse. Généralement on procède de sorte à ce que la concentration en source carbonée dans le fermenteur soit comprise entre 10 et 150 g/l, de préférence entre 35 et 80 g/l et la concentration en source azotée soit comprise entre 1 et 50 g/l de préférence entre 5 et 25 g/l.

Outre la source carbonée et la source azotée, on peut également introduire des sels minéraux nécessaires à la croissance de certains microorganismes.

Ces sels peuvent être introduits dans le fermenteur de sorte à ce que la concentration y soit comprise entre 0,001 et 1 g/l, de préférence entre 0,01 et 0,5 g/l. Les sels minéraux peuvent provenir soit du réservoir de source carbonée 3 soit du réservoir de source azotée 6.

A titre de source carbonée, on peut citer les hydrates de carbones tels que le glucose, le galactose, le fructose, la lactose, le maltose, le saccharose, l'amidon et les hydrolysats d'amidon.

A titre de source azotée, on peut utiliser des sources azotées organiques et/ou minérales. Parmi les sources azotées organiques, on peut notamment citer les proteines et les hydrolysats de protéines tels les peptones, les tryptones, la gélatine, la caséine et les caseinates, les farines animales et végétales telles les farines de mais, de soja, de blé, de poisson, le corn step liquor, la pâte de tomate, les extraits de levures. Comme source azotée minérale, on peut citer le nitrate d'ammonium, les nitrates alcalins, le chlorure d'ammonium, les carbonates de mono et diammoniques, les sulfates mono et diammoniques, les phosphates mono et diammoniques.

A titre de sels minéraux on peut citer les sulfates et les phosphates alcalins ou alcalino-terreux tels le sulfate de magnésium. Les concentrations et les différents composés indiqués ci-dessus sont utilisables pour la fabrication de bactéries lactiques, mais sont également valables d'une manière générale pour la fabrication d'autres bactéries ou même d'autres microorganismes.

Les sources carbonées et azotées en solution aqueuse sont introduites dans le fermenteur 1 par les conduites 4 et 7, sous l'action des pompes d'alimentation 5 et 8. Ces dernières sont reliées au dispositif de mesure du niveau 20 par des dispositifs électriques 21 et 22 respectivement, de sorte que lesdites pompes sont actionnées de manière à garder le volume du milieu de fermentation contenue dans le fermenteur 1, constant.

Les débits des pompes d'alimentation 5 et 8 sont réglés de manière à ce les concentrations en substrats nutritifs dans le fermenteur 1 restent constantes.

La température du milieu de fermentation est réglé au moyen d'un dispositif classique non représenté sur la figure 1. Ladite température peut être comprise entre 25 et 32°C, de préférence entre 26 et 28°C.

La pression dans le fermenteur n'est pas critique, mais est généralement choisie d'une manière conventionnelle, c'est à dire entre $10^5$ et $5.10^5$ Pa.

Au moyen de la conduite 9, on introduit dans le fermenteur 1 de l'ammoniac gazeux que l'on fait barboter dans le milieu de fermentation. L'ammoniac gazeux permet de garder le pH du milieu de fermentation à une valeur désirée. Généralement cette valeur est comprise entre 6 et 8, de préférence entre 6,3 et 7,5.

La valeur du pH est contrôlée au moyen d'un dispositif de mesure du pH 25. Eventuellement ce dispositif peut être relié au réservoir d'ammoniac gazeux de sorte à commander automatiquement l'arrivée d'ammoniac gazeux dans le milieu de fermentation, en fonction des besoins de régulation du pH.

Dans un premier temps on peut choisir de cultiver la bactérie lactique en batch. Pour se faire, on ferme les vannes 12 et 35, les vannes 17, 27 et 34 étant ouvertes.

Après la culture en batch, il peut être avantageux dans un second temps, d'éliminer au moins une partie du lactate d'ammonium formé. En effet, le lactate d'ammonium pouvant empêcher le taux de croissance d'atteindre la valeur désirée et prédéterminée. Pour se faire, les vannes 12 et 34 sont fermées et les vannes 17, 27 et 35 sont ouvertes. Le débit en milieu de fermentation 2 sortant du fermenteur 1 est réglé au moyen de la pompe 15.

Le milieu de fermentation arrive dans la cellule d'ultrafiltration 13 du coté amont 19. Le retentat est recyclé dans le fermenteur 1 par la conduite 26. Le perméat, constitué principalement d'une solution aqueuse de lactate d'ammonium, passe dans le coté aval 18, puis dans la conduite 30 et enfin dans la conduite 33, après quoi, il est éliminé.

La pompe 36 est réglée de manière à assurer un taux de dilution D suffisant pour permettre une augmentation du taux de croissance jusqu'à ladite valeur prédéterminée. A ce stade, le taux de dilution D, exprimé en $h^{-1}$, est égal au débit du perméat divisé par le volume réactionnel.

Lorsque le taux de croissance des bactéries lactiques atteint la valeur désirée, on peut commencer à purger le milieu de fermentation à un taux supérieur ou égal audit taux de croissance. Pour ce faire, on ouvre la vanne 12 et on règle le débit de la pompe 11. Le milieu de fermentation purgé sort de la conduite 10. A la sortie de cette conduite, on traite ledit milieu de fermentation purgé de sorte à récupérer les microorganismes. Un tel traitement peut consister par exemple en une congélation ou une lyophylisation.

Tout au long du procédé de fabrication des bactéries lactiques tel qu'il vient d'être décrit, on peut évaluer en permanence le taux de croissance des microorganismes d'une manière simple, ceci par détermination de la concentration en lactate d'ammonium dans le perméat. En effet, le taux de croissance $\mu$ est généralement liée à la concentration en lactate d'ammonium P selon la formule connue suivante :

$$\mu = \mu \, max \, (1 - \frac{p}{P1})^n$$

dans laquelle $\mu$ max est le taux de croissance maximun des bactéries lactiques en absence de tout inhibiteur externe au milieu de fermentation, P1 est la concentration limite en acide lactique, c'est à dire la concentration minimale en acide lactique à laquelle le taux de croissance des bactéries lactiques est nulle pour une composition donnée du milieu de culture et n est un nombre réel dépendant de la souche microbienne employée. Les valeurs de $\mu$ max, P1 et n sont déterminées de façon connue par l'homme de l'art pour chaque type de microorganisme.

La concentration en lactate d'ammonium P est connue en permanence grâce à la sonde conductimétrique 31, qui indique la valeur de cette concentration dans le perméat.

Bien que l'utilisation d'une telle sonde conductimétrique soit avantageuse, il est bien sûr possible de mesurer la concentration en lactate d'ammonium par tout autre moyen adapté à cet effet. La mesure de la concentration en lactate d'ammonium par ladite sonde conductimétrique repose sur la mesure de la conductivité du lactate d'ammonium. Il a en effet été constaté d'une manière surprenante que, d'une part, la concentration en lactacte d'ammonium est proportionnelle à la conductivité du perméat, et que d'autre part cette mesure n'était pas entravé par d'autres substances organiques présentes dans le perméat.

La figure 3 représente une courbe montrant la relation entre la concentration en lactate d'ammonium et la conductivité. Une telle mesure peut être effectuée pour des sels d'acides organiques, produits par fermentation, autres que le lactate d'ammonium.

Les microorganismes obtenues selon la présente invention présentent une activité cellulaire spécifique et une productivité cellulaire améliorée par rapport à celles des mêmes microorganismes obtenues selon les procédés de l'art antérieur.

Le procédé selon l'invention permet aussi d'obtenir des microorganismes dont l'activité cellulaire spécifique et la productivité cellulaire sont contrôlables. En effet, d'une fabrication à l'autre, il suffit de répéter les différents paramêtres, essentiellement le taux de purge, pour fabriquer des microorganismes dont les prioriétés sont très proches, voir identiques.

D'autre part, il a été constaté que le fait de purger le milieu de fermentation dans les conditions de l'invention permettait d'augmenter le rendement en biomasse formée par rapport à la source carbonée employée.

La présente invention concerne également des microorganismes susceptibles d'être obtenus selon le procédé décrit ci-dessus.

Lesdits microorganismes peuvent en particulier être des bactéries lactiques, de préférence des bactéries lactiques appartenant au genre Streptococcus.

Les conditions de réalisations préférentielles du procédé permettant de caractériser les microorganismes, deuxième objet de l'invention sont les mêmes que celle du procédé décrit plus haut, premier objet de l'invention.

Généralement, les microorganismes, en particulier les bactéries lactiques, selon l'invention ont une activité cellulaire spécifique de 20 %, plus généralement de 50 à 100 % supérieure à l'activité cellulaire spécifique de microorganismes de l'art antérieur et du même type, mais fabriqués selon les procédés de l'art antérieur.

Enfin l'invention concerne des bactéries lactiques de préférence du genre Streptococcus, dont la productivité cellulaire est supérieure à 13 UA/1/h, et généralement comprise entre 15 et 30 UA/1/h.

Les exemples suivants ont pour but d'illustrer la présente invention.

## EXEMPLES

Les exemples ci-dessous ont été réalisées à partir d'une souche Streptococcus cremoris de la société Lacto Labo dont le nom de code est Sc 301 et commercialisée par cette même société sous forme d'un levain dénommé SM 259. A l'origine, cette souche fabriquée selon un procédé classique, présente une productivité cellulaire maximum de 13 UA/1/h.

Les fermentations sont effectués dans un dispositif tel celui figurant à la figure 1.

Le fermenteur 1 a un volume utile de deux litres. La cellule d'ultrafiltration est composée de menbranes dont la surface menbranaire est égale à 0,1 m². La pompe 15 permet d'assurer la vitesse linéaire du milieu de fermentation de 5m/s. L'homogénéité du milieu de fermentation est assurée par sa recirculation. on introduit dans le dispositif de fermentation du fermenteur 1, trois litres de milieu de culture en solution aqueuse, comprenant :

– lactose : 68 g/l
– extrait de levure : 5 g/l
– tryptone : 7 g/l
– $MgSO_4$, $7H_2O$ : 0,2 g/l

Le lactose et le $MgSO_4$, $7H_2O$ proviennent du réservoir de source carbonée 3, l'extrait de levure et la tryptone proviennent du réservoir de la source azotée 6.

Au cours de la fermentation, le pH est maintenu constant à 6,3 la température à 27°C et la pression à $10^5$ Pa.

La productivité cellulaire est calculée en multipliant l'activité cellulaire spécifique par la concentration en bactérie et en divisant par le temps de fermentation. L'activité cellulaire spécifique est déterminée selon la méthode décrite plus haut.

La relation théorique qui lie le taux de croissance desdites bactéries lactiques à la concentration en lactate d'ammonium, est la suivante :
(0,9 étant le taux de croissance maximum théorique).

$$\mu = 0,9 \, (1 - P/44)$$

Toutefois, dans les conditions pratiques de réalisation décrites ci-dessus, on a pu déterminer que le taux de croissance maximun expérimental n'est pas de 0,9 mais de 0,45.

## EXEMPLE 1 (non conforme à l'invention)

On introduit dans le dispositif 3 litres de milieu de culture dont 1,5 litres proviennent du réservoir 3 et 1,5 litres du réservoir 6. On ensemence le milieu de culture avec 0,05 g de ladite souche Streptococcus cremoris.

La fermentation est d'abord réalisée en batch. Les vannes 12 et 35 sont donc fermées et les vannes 17, 27 et 34 ouvertes. Le perméat est totalement recyclé vers le fermenteur 1, après passage dans la cellule d'ultrafiltration 13 et la sonde conductimétrique 31. Le signal conductimétrique donne en permanence la concentration en lactate d'ammonium présent dans le milieu de fermentation.

Lorsque la concentration en lactate d'ammonium atteint 30 g/l, la concentration bactérienne est de 3 à 6 g/l. On ouvre la vanne 35, on ferme la vanne 34 et on règle le débit de la pompe 36 de sorte à assurer un taux de dilution D de 0,6 h⁻¹. Parallèlement, du milieu culture provenant des réservoirs 3 et 6 est introduit dans le fermenteur afin de garder constant le volume du milieu de fermentation. 17 heures après l'ouverture de la vanne 35, la concentration bactérienne est de 38 g/l. La concentration en lactate d'ammonium est de 40 g/l et le taux de croissance des bactéries est 0,08 h⁻¹. On ouvre alors la vanne 12 et on règle la pompe de purge 11 de sorte à assurer un taux de purge égal à 0,054 h⁻¹, 40 heures après la mise en place de la purge, la concentration bactérienne est de 26 g/l et celle en lactate d'ammonium est de 40,7 g/l. La productivité cellulaire des microorganismes ainsi fabriqués est de 11 UA/1/h.

## EXEMPLE 2 (conforme à l'invention)

On poursuit la fabrication de l'exemple 1 mais en diminuant la concentration en lactate jusqu'à 22 g/l. Pour ce faire, le taux de dilution D est réglé à 1,15h⁻¹. Le taux de purge est amené alors à 0,1 h⁻¹. Le volume du milieu de fermentation est toujours gardé constant par apport du milieu de culture provenant des réservoirs 3 et 6.

Le régime permanent maintenu pendant 24 heures, autorise une concentration bactérienne de 21,7 g/l et une concentration en lactate d'ammonium de 25,5 g/l. La productivité cellulaire des microorganismes obtenue est de 20 UA/1/h.

EXEMPLE 3 (conforme à l'invention)

On poursuit l'expérience de l'exemple 2 en ajustant le taux de dilution D à 0,60 h-1 et le taux de purge à 0,19 h-1. Quand le régime permanent est atteint les concentrations bactériennes et en lactate d'ammonium, sont respectivement de 11,5 g/l et 31 g/l.
La productivité cellulaire est de 26 UA/1/h.

EXEMPLE 4 (non conforme à l'invention)

Cet exemple concerne la fabrication de bactéries lactiques selon un procédé en batch.
Les conditions de réalisation de cette fabrication sont les mêmes que celles décrites pour la culture en batch conduite dans l'exemple 1. Après 14 heures de fermentation, on obtient une concentration bactérienne de 5 g/l
La productivité cellulaire des microorganismes ainsi fabriqué est de 13 UA/1/h.

EXEMPLE 5 (non conforme à l'invention)

Cet exemple concerne la fabrication de bactéries lactiques selon un procédé en recyclage total de la biomasse mettant en oeuvre une élimination des inhibiteurs de croissance par ultrafiltration. Les conditions de réalisation de cette fabrication sont les mêmes que celles décrits dans l'exemple 1, avant la mise en oeuvre de la purge, sauf que le taux de dilution D est de 0,5 h-1.
Après 90 heures de culture, on recueille les microorganismes dont la concentration dans le réacteur est de 75 g/l, et dont la productivité cellulaire est de 10 UA/1/h.

**Revendications**

**1** - Procédé de fabrication de microorganismes producteurs de leur propre inhibiteur de croissance selon lequel on fait fermenter un milieu de culture par lesdits microorganismes de manière à obtenir un milieu de fermentation, on sépare par un procédé physique de séparation au moins une partie dudit inhibiteur du milieu de fermentation, et on recycle le retentat, ledit procédé étant caractérisé en ce que lorsque le taux de croissance desdits microorganismes atteint une valeur égale à la valeur d'un taux de croissance, mesurée en régime permanent, correspondant à une activité cellulaire spécifique des microorganismes qui a été prédéterminée, on purge le milieu de fermentation selon un taux de purge supérieur ou égal audit taux de croissance.

**2** - Procédé de fabrication de microorganismes producteurs de leur propre inhibiteur de croissance, selon lequel on fait fermenter un milieu de culture par lesdits microorganismes de manière à obtenir un milieu de fermentation, on sépare par un procédé physique de séparation au moins une partie dudit inhibiteur du milieu de fermentation, et on recycle le retentat ledit prrocédé étant caractérisé en ce qu'on purge le milieu de fermentation selon un taux de purge inférieur au taux de croissance maximun théorique desdits microorganismes, de préférence le taux de purge est compris entre 10 et 80 %, plus préférentiellement entre 15 et 50 % dudit taux de croissance maximun théorique.

**3** - Procédé de fabrication de microorganismes producteurs de leur propre inhibiteur de croissance, selon lequel on fait fermenter un milieu de culture par lesdits microorganismes de manière à obtenir un milieu de fermentation, on sépare par un procédé physique de séparation au moins une parite dudit inhibiteur du milieu de fermentation, et on recycle le retentat ledit procédé étant caractérisé en ce qu'on purge le milieu de fermentation selon un taux de purge inférieur au taux de croissance maximun expérimental desdits microorganismes, de préférence le taux de purge est compris entre 15 et 80 %, plus préférentiellement entre 20 et 50 % dudit taux de croissance maximun expérimentale.

**4** - Procédé selon la revendication 1 caractérisé en ce qu'on ne purge le milieu de fermentation que lorsque ledit taux de croissance prédéterminée est atteint.

**5** - Procédé selon l'une des revendications 1 à 4 caractérisé en ce qu'avant de procéder à la purge, on fermente le milieu de culture par les microorganismes à fabriquer, en batch.

**6** - Procédé selon l'une des revendications 1 à 5 caractérisé en ce qu'avant de purger le milieu de fermentation, on sépare par un procédé physique de séparation au moins une partie dudit inhibiteur de croissance du milieu de fermentation.

**7** - Procédé selon l'une des revendications 1 à 6 caractérisé en ce que ledit procédé physique de séparation consiste en une ultrafiltration, une centrifugation, une décantation ou une dyalise, l'ultrafiltration étant préférée.

**8** - Procédé selon l'une des revendications 6 à 7 caractérisé en ce qu'on purge le milieu de fermentation en continu dès lors que la concentration en inhibiteur de croissance dans le milieu de fermentation est sensiblement constante.

**9** - Procédé selon l'une quelconque des revendications 1 à 8 caractérisé en ce que le pH du milieu de fermentation est maintenu à une valeur comprise entre 5 et 8, de préférence entre 6 et 8.

**10** - Procédé selon l'une des revendications 1 à 9 caractérisé en ce que lesdits microorganismes sont des bactéries lactiques.

**11** - Procédé selon la revendication 10 caractérisé en ce que lesdites bactéries lactiques appartiennent au genre Streptococcus.

**12** - Microorganismes susceptibles d'être obtenus selon le procédé de l'une des revendications 1 à 11.

**13** - Microorganismes selon la revendication 12 caractérisé en ce que leur activité cellulaire spécifique est de 20 %, plus généralement de 50 à 100 % supérieure à l'activité spécifique de microorganismes du même type obtenus selon un procédé de l'art antérieur.

**14** - Bactéries lactiques caractérisées en ce que leur productivité cellulaire est supérieure à 13 UA/1/h et de préférence comprise entre 15 et 30 UA/1/h.

**15** - Bactéries lactiques selon la revendication 14 caractérisées en ce que lesdites bactéries appartiennent au genre Streptococcus.

FIG.1

ACTIVITE CELLULAIRE
spécifique ( UA/g )

FIG.2

CONDUCTIVITE
(mS.cm$^{-1}$)

concentration en
acide lactique
(g.l$^{-1}$)

FIG.3

FIG.4

FIG.5

EP 0 466 560 A1

Office européen
des brevets

**RAPPORT DE RECHERCHE EUROPEENNE**

Numero de la demande

EP 91 40 1853

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | WORLD PATENT INDEX (LATEST), accession no. 86-079046 [12], semaine 12, Derwent Publications Ltd, Londres, GB; & JP-A-61 028 381 (RYOSHOKU KENKYUKAI) 08-02-1986<br>--- | 1-15 | C 12 M 1/12<br>C 12 M 1/36 |
| X,D | EP-A-0 065 895 (L'AIR LIQUIDE) * Figure 1; page 2, ligne 26 - page 4, ligne 12 *<br>--- | 1-15 | |
| A | US-A-4 167 450 (W.R. CHESBRO et al.)<br>----- | | |

DOMAINES TECHNIQUES RECHERCHES (Int. Cl.5)

C 12 M

Le présent rapport a été établi pour toutes les revendications

| Lieu de la recherche | Date d'achèvement de la recherche | Examinateur |
|---|---|---|
| LA HAYE | 22-09-1991 | TURMO Y BLANCO C.E. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
..............................................................
& : membre de la même famille, document correspondant

EPO FORM 1503 03.82 (P0402)